(19) Europäisches Patentamt
European Patent Office
Office européen des brevets

(11) Publication number: **0 173 381**
**B1**

(12) **EUROPEAN PATENT SPECIFICATION**

(45) Date of publication of patent specification: **05.04.89**

(51) Int. Cl.[4]: **B 01 J 37/04**, B 01 J 23/74, C 07 C 1/04

(21) Application number: **85201247.5**

(22) Date of filing: **29.07.85**

(54) **Catalyst mixtures.**

(30) Priority: **08.08.84 NL 8402447**

(43) Date of publication of application:
**05.03.86 Bulletin 86/10**

(45) Publication of the grant of the patent:
**05.04.89 Bulletin 89/14**

(84) Designated Contracting States:
**BE DE FR GB IT NL**

(56) References cited:
**EP-A-0 039 964**
**FR-A-2 360 531**
**NL-A-7 811 861**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Minderhoud, Johannes Kornelis**
**Badhuisweg 3**
**NL-1031 CM Amsterdam (NL)**
Inventor: **Huizinga, Tom**
**Badhuisweg 3**
**NL-1031 CM Amsterdam (NL)**
Inventor: **Sie, Swan Tiong**
**Badhuisweg 3**
**NL-1031 CM Amsterdam (NL)**

(74) Representative: **Aalbers, Onno et al**
**P.O. Box 302**
**NL-2501 CH The Hague (NL)**

**EP 0 173 381 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to novel catalyst mixtures as well as to the use of these catalyst mixtures in the preparation of hydrocarbon mixtures from mixtures of carbon monoxide and hydrogen.

Mixtures of carbon monoxide and hydrogen can be converted into hydrocarbon mixtures by using a mixture of two catalysts, one being capable of catalysing the conversion of a $H_2/CO$ mixture into oxygen-containing organic compounds and the other being a crystalline iron silicate of a special structure, which is capable of catalysing the conversion of organic oxygen compounds into a mixture of hydrocarbons. Said crystalline iron silicates are characterized in that, after one hour's calcination in air at 500°C, they have the following properties:

a) an X-ray powder diffraction pattern in which the strongest lines are the four lines mentioned in Table A, and

TABLE A

| d(Å) |
|---|
| 11.1 ±0.2 |
| 10.1 ±0.2 |
| 3.84±0.07 |
| 3.72±0.06 |

b) in the formula which represents the composition of the silicate, expressed in moles of the oxides, the $SiO_2/Fe_2O_3$ molar ratio is 20—2000.

The crystalline iron silicates which are used in the catalyst mixtures can be prepared starting from an aqueous mixture comprising the following compounds: one or more compounds of an alkali metal (M), one or more organic nitrogen compounds (RN), one or more silicon compounds and one or more compounds containing iron in a trivalent form. The preparation of the crystalline iron silicates is carried out by keeping the mixture at an elevated temperature until the crystalline silicate has formed, separating the latter from the mother liquor, and calcining.

When the above-mentioned catalyst mixtures are used for the preparation of hydrocarbon mixtures from $H_2/CO$ mixtures, they show high activity, very high selectivity towards hydrocarbons, very high $C_3^+$ selectivity as related to $C_1^+$ and good stability. However, the hydrocarbon mixtures obtained have a relatively low aromatics content. In those cases where the preparation of hydrocarbon mixtures with a relatively high aromatics content is desired, the above-mentioned catalyst mixture proves inadequate.

Besides the afore-described crystalline iron silicates, there are also known crystalline boron silicates, which have the same special structure as the iron silicates. These boron silicates can be prepared in substantially the same manner as the iron silicates, the difference being that in the aqueous mixture from which the iron silicates are prepared, the trivalent iron compound is replaced by a boron compound.

In view of the fact that for a number of catalytic uses, such as the isomerization of mixtures of $C_8$ aromatics for the preparation of para-xylene, the boron silicates show the same performance, quality-wise, as the iron silicates, experiments were carried out in order to establish whether the demand for higher aromatics selectivity could be met by substituting in the catalyst mixtures a boron silicate for the iron silicate. The results of these experiments are very disappointing. As compared to the catalyst mixtures containing an iron silicate, those in which a boron silicate was present showed considerably lower aromatics selectivities. And also in a number of other areas did the catalyst mixtures containing a boron silicate prove altogether deficient. They showed very low activities, low selectivities towards hydrocarbons, and considerably lower $C_3^+$ selectivities in relation to $C_1^+$ and stabilities than the catalyst mixtures containing an iron silicate. By reason of these results the crystalline boron silicates must be regarded as totally unfit for use as components in the catalyst mixtures for the preparation of hydrocarbon mixtures from $H_2/CO$ mixtures.

In the face of the disappointing results obtained by using the catalyst mixtures containing a crystalline boron silicate, the investigation was continued using catalyst mixtures containing a crystalline silicate in which boron was present in addition to iron. Such silicates, which have the same special structure as the afore-mentioned iron silicates and boron silicates, can be obtained in a simple manner starting from an aqueous mixture which, in addition to the other compounds required for preparing silicates of the desired special structure, contains both a trivalent iron compound and a boron compound. This has surprisingly led to the finding that catalyst mixtures wherein the crystalline silicate component of a special structure is a silicate which, in addition to a given quantity of iron, contains a given quantity of boron, show excellent performance in the preparation of hydrocarbon mixtures from $H_2/CO$ mixtures. In comparison with the catalyst mixtures containing an iron silicate, those containing an iron/boron silicate show a considerably higher aromatics selectivity, whilst their activity, selectivity to hydrocarbons, $C_3^+$ selectivity as related to $C_1^+$ and stability are on an equally high level as for the catalyst mixtures containing an iron silicate. In order for this favourable performance to be achieved it is necessary that in the formula which represents the composition of the silicate used, expressed in moles of the oxides, the $SiO_2/Fe_2O_3$ molar ratio is 20—2000, the $SiO_2/B_2O_3$ molar ratio is 50—5000, and the $Fe_2O_3/B_2O_3$ molar ratio is higher than 1.0.

The above-described catalyst mixtures containing an iron-boron silicate are novel compositions.

The present patent application therefore relates to novel catalyst mixtures composed of two catalysts, one being capable of catalysing the conversion of a $H_2/CO$ mixture into oxygen-containing organic compounds, and the other being a crystalline iron/boron silicate which, after one hour's calcination in air at 500°C, has the following properties:

a) an X-ray powder diffraction pattern in which the strongest lines are the four lines mentioned in Table A, and

b) in the formula which represents the composition of the silicate, expressed in moles of the oxides, the $SiO_2/Fe_2/O_3$ molar ratio is 20—2000, the $SiO_2/B_2O_3$ molar ratio is 50—5000 and the $Fe_2O_3/B_2O_3$ molar ratio is higher than 1.0.

The patent application further relates to the use of these catalyst mixtures in the preparation of hydrocarbon mixtures from $H_2/CO$ mixtures.

One of the two catalysts in the catalyst mixture according to the invention is a catalyst which is capable of catalysing the conversion of a $H_2/CO$ mixture into oxygen-containing organic compounds. The catalyst mixture preferably comprises a catalyst which is capable of converting a $H_2/CO$ mixture substantially into methanol and/or dimethyl ether. Very suitable are zinc-containing compositions which, in addition to zinc, comprise one or more of the metals chromium, copper and aluminium. Examples of suitable metal combinations are zinc-chromium, zinc-chromium-copper and zinc-aluminium-copper. Preference is given to the use of catalysts which, in addition to zinc, comprise chromium, in particular catalyst in which the atomic percentage of zinc, calculated on the sum of zinc and chromium, is at least 60% and in particular 60—80%. The above-mentioned zinc-containing compositions are usually prepared by calcining one or more precipitates which have been obtained by the addition of a basic reacting substance to one or more aqueous solutions containing salts of the metals concerned. Starting from the zinc-containing compositions thus prepared, the catalyst mixtures according to the invention can be obtained by mechanically mixing the particles of the zinc-containing composition and the crystalline iron/boron silicate. Another very attractive method of preparing the catalyst mixtures according to the invention is spray drying. Spray drying is a procedure for preparing small globular particles starting from a solid material or a mixture of solids, which has been in use on a commercial scale for many years now. It is carried out by vaporizing a dispersion in water of the material to be spray dried through a nozzle or from a rotating disc into a hot gas. It is a very suitable procedure for bringing about a very intimate contact between different substances. The preparation of the present catalyst mixtures by spray drying can be carried out by dispersing the crystalline iron/boron silicate in water, together with one or more precipitates in which zinc and one or more metals chosen from chromium, copper and aluminium are present, which precipitates have been obtained by adding a basic reacting substance to one or more aqueous solutions of salts of the metals concerned, and spray drying the dispersion thus obtained to prepare the desired catalyst mixture. In view of their form, size and strength, the catalyst particles prepared by spray drying are very suitable for use in a fluidized state.

As regards the ratio in which the two catalysts are present in the catalyst mixtures according to the invention, it is preferred that per part by weight of the crystalline iron/boron silicate catalyst, the catalyst mixtures should comprise 2.5—12.5 pbw of the catalyst having activity for the conversion of a $H_2/CO$ mixture.

The crystalline iron/boron silicates used as catalyst components in the catalyst mixtures according to the invention are defined, among other things, with the aid of the X-ray powder diffraction pattern which they display after one hour's calcination in air at 500°C. In this pattern the strongest lines should be the four lines mentioned in Table A. The complete X-ray powder diffraction pattern of a typical example of the present crystalline iron/boron silicates after one hour's calcination in air at 500°C, is given in Table B.

TABLE B

| d(Å) | Rel. int. | d(Å) | Rel. int. |
|---|---|---|---|
| 11.1 | 100 | 3.84 (D) | 57 |
| 10.0 (D) | 70 | 3.72 (D) | 31 |
| 8.93 | 1 | 3.63 | 16 |
| 7.99 | 1 | 3.47 | <1 |
| 7.42 | 2 | 3.43 | 5 |
| 6.68 | 7 | 3.34 | 2 |
| 6.35 | 11 | 3.30 | 5 |
| 5.97 | 17 | 3.25 | 1 |
| 5.70 | 7 | 3.05 | 8 |
| 5.56 | 10 | 2.98 | 11 |
| 5.35 | 2 | 2.96 | 3 |
| 4.98 (D) | 6 | 2.86 | 2 |
| 4.60 | 4 | 2.73 | 2 |
| 4.35 | 5 | 2.60 | 2 |
| 4.25 | 7 | 2.48 | 3 |
| 4.07 | 2 | 2.40 | 2 |
| 4.00 | 4 | | |

(D)=doublet

The crystalline iron/boron silicates which are used in the catalyst mixtures according to the invention can be prepared starting from an aqueous solution comprising the following compounds: one or more compounds of an alkali metal (M), one or more organic nitrogen compounds (RN), one or more silicon compounds, one or more compounds in which iron is present in a trivalent form, and one or more boron compounds. The preparation is carried out by keeping the mixture at an elevated temperature until the silicate has formed, and subsequently separating the silicate crystals from the mother liquor. In the aqueous solution from which the silicates are prepared the various compounds should be present in the following molar ratios, expressed—with the exception of the organic nitrogen compounds—as moles of the oxides:

$M_2O:SiO_2=0.01—0.35$,
$RN:SiO_2=0.04—2.0$,
$SiO_2:Fe_2O_3=30—3000$,
$SiO_2:B_2O_3=50—5000$, and
$H_2O:SiO_2=5—100$.

The preparation of the silicates may be carried out either at atmospheric pressure or at an elevated pressure. If reaction temperatures are used which lie above the boiling point of the mixture it is preferred to use an autoclave under autogenous pressure. The silicates are preferably prepared by keeping the mixture for at least four hours at a temperature between 125 and 175°C. After the formation of the silicates the crystals are separated from the mother liquor, for instance by filtration, decantation or centrifugation. The crystal mass is subsequently washed with water and finally dried at a temperature between 100 and 200°C.

Examples that may be mentioned of suitable compounds which can be used in the preparation of the iron/boron silicates are nitrates, carbonates, hydroxides and oxides of alkali metals; primary, secondary

and tertiary alkyl amines and quaternary alkyl ammonium compounds, such as bromides and hydroxides; heterocyclic nitrogen compounds, such as pyridine and piperidine; sodium silicate, silicic acid and amorphous silica; boric acid and borax; the nitrate, sulphate, chloride and hydroxide of trivalent iron. In the preparation of the crystalline iron/boron silicates it is preferred to start from a base mixture in which M is present in a sodium compound and which comprises butylamine as RN compound, amorphous silica a silicon compound and boric acid as boron compound. In the crystalline iron/boron silicates used in the catalyst mixtures according to the invention the $SiO_2/Fe_2O_3$ molar ratio should be 20—2000 and the $SiO_2/B_2O_3$ molar ratio 50—5000. Preferably use is made of crystalline iron/boron silicates which have a $SiO_2/Fe_2O_3$ molar ratio of 50—300 and a $SiO_2/B_2O_3$ molar ratio of 100—1000.

The silicates prepared as described hereinabove contain alkali metal ions. By using suitable exchange methods these can be replaced by other cations, such as hydrogen ions or ammonium ions. The crystalline iron/boron silicates which are used in the present catalyst mixtures preferably have an alkali metal content of less than 0.05 %w.

As stated hereinbefore, the present patent application also relates to the use of the catalyst mixtures according to the invention in the preparation of hydrocarbon mixtures from $H_2/CO$ mixtures. Suitable $H_2/CO$ mixtures can be prepared by gasification of heavy carbonaceous materials such as coal, or by steam reforming or partial oxidation of light hydrocarbons such as natural gas. Suitable conditions for the conversion of $H_2/CO$ mixtures into hydrocarbon mixtures according to the invention are a temperature of 200—500°C and in particular of 300—450°C, a pressure of 1—150 bar ($10^5$—$1.5 \cdot 10^7$ Pa) and in particular of 5—100 bar ($5 \cdot 10^5$—$10^7$ Pa) and a space velocity of 50—5000 and in particular 300—3000 $Nl \cdot l^{-1} \cdot h^{-1}$.

The above-described process, in which a hydrocarbon mixture is prepared from a $H_2/CO$ mixture can very suitably be carried out as an individual process in which the conversion of the $H_2/CO$ mixture is performed in a single step. Unconverted synthesis gas can be recirculated, if desired. The process can also very suitably be carried out as part of a multi-step process for the conversion of $H_2/CO$ mixtures into hydrocarbon mixtures, in which case two options offer, viz.

a) the process is carried out as the first step of a two-step process in which $H_2$ and CO present in the reaction product from the first step—optionally together with other components from this reaction product—is contacted in a second step with a catalyst comprising one or more metal components which have activity for the conversion of a $H_2/CO$ mixture into paraffinic hydrocarbons, and which have been chosen from the group formed by cobalt, nickel and ruthenium,

b) the process is carried out as the first step of a three-step process in which the first two steps are conducted as described under a) the catalyst used in the second step being a zirconium-, titanium- or chromium-promoted cobalt catalyst supported on silica, alumina or silica-alumina as carrier, which catalyst has been prepared by impregnation and/or kneading. In this three-step process advantage is taken from the fact that by using a catalytic hydrocracking the high-boiling part of the reaction product from the second step can be converted in high yield into middle distillates.

The three-step process mentioned under b) involves carrying out an additional catalytic hydrocracking as a third step following the two-step process as mentioned under a). As feed for the catalytic hydrocracking treatment is chosen at least the part of the reaction product from the second step of which the initial boiling point lies above the final boiling point of the heaviest middle distillate desired as end product. The hydrocracking treatment, which is characterized by a very low hydrogen consumption, yields middle distillates which have a considerably lower pour point than those obtained by the direct conversion of a $H_2/CO$ mixture according to Fischer-Tropsch. Catalysts which are very suitable for carrying out the catalytic hydrocracking are those comprising one or more noble metals from Group VIII supported on a carrier.

The invention is now illustrated with the aid of the following example.

Example

Three crystalline silicates (silicates 1—3) were prepared by maintaining mixtures of amorphous silica, sodium hydroxide, butylamine and, either ferric nitrate, or boric acid, or both ferric nitrate and boric acid, in water, in an autoclave under autogenous pressure and with stirring, for 120 hours at 150°C. After cooling of the reaction mixtures the silicates were filtered off, washed with water until the pH of the wash water was about 8, dried at 120°C and calcined in air for one hour at 500°C. The silicates 1—3 had the following properties

a) an X-ray powder diffraction pattern substantially corresponding with that mentioned in Table B, and

b) an $SiO_2/Fe_2O_3$ molar ratio of 100 for iron silicate 1, an $SiO_2/B_2O_3$ molar ratio of 200 for boron silicate 2, and an $SiO_2/Fe_2O_3$ molar ratio of 120 and an $SiO_2/B_2O_3$ molar ratio of 215 for iron/boron silicate 3.

The molar composition of the aqueous mixtures from which silicates 1—3 were prepared may be rendered as follows

$$1.0\ Na_2O \cdot 25\ SiO_2 \cdot 10\ C_4H_9NH_2 \cdot x\ Fe_2O_3 \cdot y\ B_2O_3 \cdot 450\ H_2O,$$

wherein x and y have the following values

silicate 1 x=0.2; y=0
silicate 2 x=0; y=0.1
silicate 3 x=0.2; y=0.1.

From silicates 1—3 were prepared silicates I—III, respectively, by boiling silicates 1—3 with a 1.0 molar $NH_4NO_3$ solution, washing with water, boiling again with a 1.0 molar $NH_4NO_3$ solution and washing, drying at 120°C and calcining at 500°C. Subsequently three catalyst mixtures (catalyst mixtures A—C) were prepared by mixing a $ZnO—Cr_2O_3$ composition with each one of silicates I—III. The Zn atomic percentage of the $ZnO—Cr_2O_3$ composition was 70%. All the catalyst mixtures contained per part by weight of silicate 10 parts by weight of the $ZnO—Cr_2O_3$ composition. Catalyst mixtures A—C were tested for suitability in the preparation of hydrocarbon mixtures from a $H_2/CO$ mixture. The testing was carried out in a 50 ml reactor containing a fixed catalyst bed of 7.5 ml volume. In three experiments a $H_2/CO$ mixture of a $H_2/CO$ molar ratio of 0.5 was passed over each one of catalyst mixtures A—C at a temperature of 375°C, a pressure of 60 bar ($6 \cdot 10^6$ Pa) and a space velocity of 850 $Nl \cdot kg^{-1} \cdot h^{-1}$. The results of these experiments, measured at run hour 50, are given in Table C.

Of the catalyst mixtures and experiments mentioned in Table C only catalyst mixture C and experiment 3 are according to the invention. Catalyst mixtures A and B and experiments 1 and 2 fall outside the scope of the invention. They have been included in the patent application for comparison.

TABLE C

| Experiment No. | 1 | 2 | 3 |
|---|---|---|---|
| Catalyst mixture | A | B | C |
| Silicate No. | I | II | III |
| ($H_2$+CO) conversion, %v | 59 | 19 | 57 |
| Selectivity towards hydrocarbons, %w | 100 | 66 | 100 |
| $C_3^+$ selectivity, %w on $C_1^+$ | 94 | 81 | 93 |
| Aromatics, %w on $C_1^+$ | 20 | 15 | 26 |
| Loss of ($H_2$+CO) conversion, %v per 100 h | 5 | 10 | 5 |

**Claims**

1. Catalyst mixtures consisting of two catalysts, characterized in that one is capable of catalysing the conversion of a $H_2/CO$ mixture into oxygen-containing organic compounds, and the other is a crystalline iron/boron silicate which, after one hour's calcination in air at 500°C, has the following properties

a) an X-ray powder diffraction pattern in which the strongest lines are the four lines mentioned in Table A, and

TABLE A

| d(Å) |
|---|
| 11.1 ±0.2 |
| 10.0 ±0.2 |
| 3.84±0.07 |
| 3.72±0.06 |

b) in the formula which represents the composition of the silicate, expressed in moles of the oxides, the $SiO_2/Fe_2O_3$ molar ratio is 20—2000, the $SiO_2/B_2O_3$ molar ratio 50—5000 and the $Fe_2O_3/B_2O_3$ molar ratio higher than 1.0.

2. Catalyst mixtures as claimed in claim 1, characterized in that the catalyst present in the catalyst mixture which is capable of catalysing the conversion of a $H_2/CO$ mixture into oxygen-containing organic compounds is a zinc-containing composition which, in addition to zinc, comprises one or more of the metals chromium, copper and aluminium.

3. Catalyst mixtures as claimed in claim 2, characterized in that, in addition to zinc, the zinc-containing composition comprises chromium and that the atomic percentage of zinc, calculated on the sum of zinc and chromium, is at least 60%.

4. Catalyst mixtures as claimed in any one of claims 1—3, characterized in that the silicate present therein has been prepared by maintaining an aqueous mixture comprising the following compounds: one or more compounds of an alkali metal (M), one or more organic nitrogen compounds (RN), one or more silicon compounds, one or more compounds in which iron is present in a trivalent form and one or more

boron compounds, in which mixture the various compounds are present in the following molar ratios, expressed—with the exception of the organic nitrogen compounds—in moles of the oxides:

$M_2O:SiO_2$=0.01—0.35,
$RN:SiO_2$=0.04—2.0,
$SiO_2:Fe_2O_3$=30—3000,
$SiO_2:B_2O_3$=50—5000, and
$H_2O:SiO_2$=5—100

at an elevated temperature until the crystalline silicate has formed, which is subsequently separated from the mother liquor and calcined.

5. Catalyst mixtures as claimed in claim 4, characterized in that in the preparation of the silicate a sodium compound is used as alkali metal compound, butylamine as RN compound, amorphous silica as silicon compound and boric acid as boron compound.

6. Catalyst mixtures as claimed in any one of claims 1—5, characterized in that the silicate present therein has an $SiO_2/Fe_2O_3$ molar ratio of 50—300 and an $SiO_2/B_2O_3$ molar ratio of 100—1000.

7. Catalyst mixtures as claimed in any one of claims 1—6, characterized in that the silicate present therein has an alkali metal content lower than 0.05 %w.

8. Catalyst mixtures as claimed in any one of claims 1—7, characterized in that per part by weight of the crystalline silicate catalyst they comprise 2.5—12.5 parts by weight of the catalyst with activity for the conversion of the $H_2/CO$ mixture.

9. Catalyst mixtures as claimed in any one of claims 1—8, characterized in that they have been prepared by spray drying.

10. A process for the preparation of a hydrocarbon mixture, characterized in that a $H_2/CO$ mixture is contacted with a catalyst mixture as claimed in claim 1.

11. A process as claimed in claim 10, characterized in that the $H_2/CO$ mixture has been obtained by gasification of a heavy carbonaceous material such as coal, or by steam reforming or partial oxidation of light hydrocarbons such as natural gas.

12. A process as claimed in claim 10 or 11, characterized in that it is carried out at a temperature of 200—500°C, a pressure of $10^5$—$1.5\times10^7$ Pa (1—150 bar) and a space velocity of 50—5000 $Nl \cdot l^{-1} \cdot h^{-1}$.

13. A process as claimed in claim 12, characterized in that it is carried out at a temperature of 300—450°C, a pressure of $5\times10^5$—$10^7$ Pa (5—100) and a space velocity of 300—3000 $Nl \cdot l^{-1} \cdot h^{-1}$.

14. A process as claimed in any one of claims 10—13, characterized in that it is used as the first step of a two-step process, that $H_2$ and CO present in the reaction product from the first step—together with other components of this reaction product, if desired—is contacted in the second step with a catalyst comprising one or more metal components having activity for the conversion of a $H_2/CO$ mixture into paraffinic hydrocarbons, which metal components have been chosen from the group formed by cobalt, nickel and ruthenium.

15. A process as claimed in any one of claims 10—13, characterized in that it is used as the first step of a three-step process, that the first two steps are carried out as stated in claim 14, that in the second step used is made of a zirconium-, titanium- or chromium-promoted cobalt catalyst supported on silica, alumina or silica-alumina as carrier, which catalyst has been prepared by impregnation and/or kneading, and that middle distillates are prepared by separating from the reaction product from the second step at least the part whose initial boiling point lies above the final boiling point of the heaviest middle distillate desired as end product and subjecting it in the third step to a catalytic hydrocracking treatment.

**Patentansprüche**

1. Katalysatormischungen bestehend aus zwei Katalysatoren, dadurch gekennzeichnet, daß einer davon dazu in der Lage ist, die Umwandlung einer $H_2/CO$-Mischung in sauerstoffhaltige organische Verbindungen zu katalysieren, und der andere ein kristallines Eisen/Bor-Silikat ist, welches nach einstündigem Kalzinieren an Luftbei 500°C die folgenden Eigenschaften aufweist:

a) ein Röntgenbeugungspulverdiagramm, in welchem die stärksten Linien die in Tabelle A genannten vier Linien sind.

TABELLE A

| d(Å) |
|---|
| 11,1 ±0,2 |
| 10,0 ±0,2 |
| 3,84±0,07 |
| 3,72±0,06 |

b) in der Formel, welche die Zusammensetzung des Silikats wiedergibt, ausgedrückt in Mol der Oxide,

ist das molare Verhältnis von $SiO_2/Fe_2O_3$ im Bereich von 20:1 bis 2000:1, das molare Verhältnis von $SiO_2/B_2O_3$ im Bereich von 50:1 bis 5000:1 und das molare Verhältnis von $Fe_2O_3/B_2O_3$ liegt über 1,0:1.

2. Katalysatormischungen wie in Anspruch 1 beansprucht, dadurch gekennzeichnet, daß der in der Katalysatormischung vorliegende Katalysator, welcher dazu in der Lage ist, die Umwandlung einer $H_2$/CO-Mischung in sauerstoffhaltige organische Verbindungen zu katalysieren, eine Zink enthaltende Zusammensetzung ist, welche zusätzlich zu Zink eines oder mehrere der Metalle Chrom, Kupfer und Aluminium enthält.

3. Katalysatormischungen wie in Anspruch 2 beansprucht, dadurch gekennzeichnet, daß die zinkhaltige Zusammensetzung außer Zink Chrom enthält und daß der Atomanteil an Zink, mindestens 60% beträgt, bezogen auf die Summe aus Zink und Chrom.

4. Katalysatormischungen, wie in einem der Ansprüche 1 bis 3 beansprucht, dadurch gekennzeichnet, daß das darin enthaltene Silikat hergestellt wurde, indem man eine wäßrige Mischung, umfassend die folgenden Verbindungen: eine oder mehrere Verbindungen eines Alkalimetalls (M), eine oder mehrere organische Stickstoffverbindungen (RN), eine oder mehrer Siliciumverbindungen, eine oder mehrere Verbindungen, in welchen Eisen in dreiwertiger Form enthalten ist, und eine oder mehrere Borverbindungen, in welcher Mischung die verschiedenen Verbindungen in den folgenen Molverhältnissen vorliegen, ausgedrückt in Mol der Oxide—mit Ausnahme der organischen Stickstoffverbindungen:

$M_2O:SiO_2=0,01:1—0,35:1;$
$RN:SiO_2=0,04:1—2,0:1;$
$SiO_2:Fe_2O_3=30:1—3000:1;$
$SiO_2:B_2O_3=50:1—5000:1$, und
$H_2O:SiO_2=5:1—500:1.$

auf erhöhter Temperatur hielt, bis das kristalline Silikat sich gebildet hat, welches anschließend von der Mutterlauge abgetrennt und calziniert wurde.

5. Katalysatormischungen wie in Anspruch 4 beansprucht, dadurch gekennzeichnet, daß für die Herstellung des Silikats eine Natriumverbindung als Alkalimetallverbindung eingesetzt wird, Butylamin als RN-Verbindung, amorphes Siliciumdioxid als Silicium verbindung und Borsäure als Borverbindung eingesetzt wird.

6. Katalysatormischungen wie in einem der Ansprüche 1 bis 5 beansprucht, dadurch gekennzeichnet, daß das darin enthaltene Silikat ein molares Verhältnis von $SiO_2/Fe_2O_3$ von 50:1 bis 300:1 und ein molares Verhältnis von $SiO_2/B_2O_3$ von 100:1 bis 1000:1 aufweist.

7. Katalysatormischungen wie in einem der Ansprüche 1 bis 6 beansprucht, dadurch gekennzeichnet, daß das darin enthaltene Silikat einen Alkalimetallgehalt von weniger als 0,05 Gewichtsprozent aufweist.

8. Katalysatormischungen wie in einem der Ansprüche 1 bis 7 beansprucht, dadurch gekennzeichnet, daß pro Gewichtsteil des kristallinen Silikatkatalysators 2,5 bis 12,5 Gewichtsteile des Katalysators, der die Wirkung für die Umwandlung der $H_2$/CO-Mischung besitzt, darin enthalten sind.

9. Katalysatormischungen wie in einem der Ansprüche 1 bis 8 beansprucht, dadurch gekennzeichnet, daß sie durch Sprühtrocknung hergestellt wurden.

10. Ein Verfahren zur Herstellung einer Kohlenwasserstoffmischung, dadurch gekennzeichnet, daß eine $H_2$/CO-Mischung mit einer Katalysatormischung, wie in Anspruch 1 beansprucht, kontaktiert wird.

11. Ein Verfahren wie in Anspruch 10 beansprucht, dadurch gekennzeichnet, daß die $H_2$/CO-Mischung durch Vergasung von schwerem kohlenstoffhaltigen Material, wie z.B. Kohle, oder durch Dampfreformierung oder teilweise Oxidation von leichten Kohlenwasserstoffen, wie z.B. Erdgas, erhalten wurde.

12. Ein Verfahren wie in Anspruch 10 oder 11 beansprucht, dadurch gekennzeichnet, daß es bei einer Temperatur von 200 bis 500°C, einem Druck von $10^5$—$1{,}5\times10^7$ Pa (1—150 bar) und einer Raumgeschwindigkeit von 50—5000 $Nl \cdot l^{-1} \cdot h^{-1}$ durchgeführt wird.

13. Ein Verfahren wie in Anspruch 12 beansprucht, dadurch gekennzeichnet, daß es bei einer Temperatur von 300 bis 450°C, eimem Druck von $5\times10^5$—$10^7$ Pa (5—100 bar) und einer Raumgeschwindigkeit von 300—3000 $Nl \cdot l^{-1} \cdot h^{-1}$ durchgeführt wird.

14. Ein Verfahren wie in einem der Ansprüche 10 bis 13 beansprucht, dadurch gekennzeichnet, daß es als erste Stufe eines Zweistufenverfahrens eingesetzt wird, daß $H_2$ und CO, die im Reaktionsprodukt aus der ersten Stufe vorliegen—zusammen mit anderen Komponenten dieses Reaktionsproduktes, falls erwünscht—in der zweiten Stufe mit einem Katalysator kontaktiert werden, welcher ein oder mehrere Metallkomponenten, welche eine entsprechende Aktivität für die Umwandlung einer $H_2$/CO-Mischung in paraffinische Kohlenwasserstoffe aufweisen und welche aus der Gruppe, gebildet aus Kobalt, Nickel und Ruthenium, ausgewählt worden sind, enthält.

15. Ein Verfahren wie in einem der Ansprüche 10 bis 13 beansprucht, dadurch gekennzeichnet, daß es als erste Stufe eines Dreistufenverfahrens eingesetzt wird, daß die ersten beiden Stufen durchgeführt werden wie in Anspruch 14 beschrieben, daß in der zweiten Stufe ein Zirkonium, Titan oder Chrom als Promotor enthaltender Kobaltkatalysator auf einem Siliciumdioxidträger, einem Aluminiumoxid- oder einem Siliciumdioxid - Aluminiumoxidträger eingesetzt wird, welcher Katalysator durch Impregnation und/oder Kneten hergestellt worden ist, und daß Mitteldestillate durch Abtrennen aus dem Reaktionsprodukt der zweiten Stufe mindestens des Teils, dessen Anfangssiedepunkt oberhalb des Endsiedepunktes

des schwersten Mitteldestillates liegt, das als Endprodukt gewünscht wird, und Durchführen einer katalytischen Hydrocrackbehandlung mit diesem Teil in der dritten Stufe hergestellt werden.

**Revendications**

Mélanges catalytiques constitués de deux catalyseurs, caractérisés en ce que l'un est capable de catalyser la conversion d'un mélange $H_2/CO$ en composés organiques contenant de l'oxygène et l'autre est un silicate cristallin de fer/bore qui, après calcination pendant une heure dans l'air à 500°C, a les propriétés suivantes

a) un diagramme de diffraction des rayons X par la méthode des poudres dans lequel les lignes les plus intenses sont les quatre lignes mentionnées dans le tableau A

TABLEAU A

| d(Å) |
|---|
| 11,1 ±0,2 |
| 10,0 ±0,2 |
| 3,84±0,07 |
| 3,72±0,06 |

b) dans la formule qui représente la composition du silicate, exprimée en moles des oxydes, le rapport molaire $SiO_2/Fe_2O_3$ est de 20—2000, le rapport molaire $SiO_2/B_2O_3$ est de 50—5000 et le rapport molaire $Fe_2O_3/B_2O_3$ est supérieur à 1,0.

2. Mélanges catalytiques selon la revendication 1, caractérisés en ce que le catalyseur présent dans le mélange catalytique qui est capable de catalyser la conversion d'un mélange $H_2/CO$ en composés organiques contenant de l'oxygène est une composition contenant du zinc qui, en plus du zinc, comprend un ou plusieurs des métaux chrome, cuivre et aluminium.

3. Mélanges catalytiques selon la revendication 2, caractérisés en ce que, en plus du zinc, la composition contenant du zinc comprend du chrome, et que le pourcentage atomique de zinc, calculé par rapport à la somme du zinc et du chrome, est d'au moins 60%.

4. Mélanges catalytiques selon l'une quelconque des revendications 1—3, caractérisés en ce que le silicate qui s'y trouve présent a été préparé en maintenant un mélange aqueux comprenant les composés suivants: un ou plusieurs composés d'un métal alcalin (M), un ou plusieurs composés organiques azotés (RN), un ou plusieurs composés du silicium, un ou plusieurs composés dans lesquels du fer est présent dans une forme trivalente et un ou plusieurs composés du bore, mélange dans lequel les divers composés sont pésents dans les rapports molaires suivants, exprimés—à l'exception des composés organiques azotés—en moles des oxydes:

$M_2O:SiO_2$=0,01—0,35,
$RN:SiO_2$=0,04—2,0,
$SiO_2:Fe_2O_3$=30—3000,
$SiO_2:B_2O_3$=50—5000, et
$H_2O:SiO_2$=5—100

à une température élevée jusqu'à ce que le silicate cristallin soit formé, ce dernier étant ensuite séparé de la liqueur-mère et calciné.

5. Mélanges catalytiques selon la revendication 4, caractérisés en ce que dans la préparation du silicate on utilise un composé du sodium comme composé de métal alcalin, la butylamine comme composé RN, de la silice amorphe comme composé du silicium et l'acide borique comme composé du bore.

6. Mélange catalytiques selon l'une quelconque des revendications 1—5, caractérisés en ce que le silicate qui s'y trouve présent a un rapport molaire $SiO_2/Fe_2O_3$ de 50—300 et un rapport molaire $SiO_2/B_2O_3$ de 100—1000.

7. Mélanges catalytiques selon l'une quelconque des revendications 1—6, caractérisés en ce que le silicate qui s'y trouve présent a une teneur en métaux alcalins inférieure à 0,05% en poids.

8. Mélanges catalytiques selon l'une quelconque des revendications 1—7, caractérisés en ce que, par partie en poids du catalyseur silicate cristallin, ils comprennent 2,5—12,5 parties en poids du catalyseur ayant une activité pour la conversion du mélange $H_2/CO$.

9. Mélanges catalytiques selon l'une quelconque des revendications 1—8, caractérisés en ce qu'ils ont été préparés par séchage par pulvérisation.

10. Un procédé pour la préparation d'un mélange d'hydrocarbures, caractérisé en ce qu'un mélange $H_2/CO$ est mis en contact avec un mélange catalytique selon la revendication 1.

11. Un procédé selon la revendication 10, caractérisé en ce que le mélange $H_2/CO$ a été obtenu par gazéification d'une matière carbonée lourde telle que du charbon, ou par reformage à la vapeur d'eau ou oxydation partielle d'hydrocarbures légers comme du gaz naturel.

12. Un procédé selon la revendication 10 ou 11, caractérisé en ce qu'il est mis en oeuvre à une température de 200—500°C, une pression de $10^5$—$1,5\times10^7$ Pa (1—150 bars) et une vitesse spatiale de 50—5000 litres (TPN) par litre et par heure.

13. Un procédé selon la revendication 12, caractérisé en ce qu'il est mis en oeuvre à une température de 300—450°C, une pression de $5\times10^5$—$10^7$ Pa (5—100 bars) et une vitesse spatiale de 300—3000 litres (TPN) par litre et par heure.

14. Un procédé selon l'une quelconque des revendications 10—13, caractérisé en ce qu'il est utilisé comme première étape d'un procédé à deux étapes, que $H_2$ et CO présents dans le produit de réaction de la première étape—en même temps que d'autres constituants de ce produit de réaction, si on le désire—sont mis en contact dans la seconde étape avec un catalyseur comprenant un ou plusieurs constituants métalliques ayant une activité pour la conversion d'un mélange $H_2$/CO en hydrocarbures paraffiniques, ces constituants métalliques ayant été choisis dans le groupe formé par le cobalt, le nickel et le ruthénium.

15. Un procédé selon l'une quelconque des revendications 10—13, caractérisé en ce qu'il est utilisé comme première étape d'un procédé à trois étapes, que les deux premières étapes sont conduites comme spécifié dans la revendication 14, que dans la seconde étape on utilise un catalyseur au cobalt activé par du zirconium, du titane ou du chrome déposé sur de la silice, de l'alumine ou une combinaison silice-alumine comme support, ce catalyseur ayant été préparé par imprégnation et/ou malaxage, et que l'on prépare des distillats moyens en séparant du produit de réaction de la seconde étape au moins la partie dont le point initial d'ébullition se trouve au-dessus du point final d'ébullition du distillat moyen le plus lourd désiré comme produit final et en la soumettant dans la troisième étape à un traitement d'hydrocraquage catalytique.